(19) Europäisches Patentamt
European Patent Office
Office européen des brevets

(11) **EP 3 933 551 A1**

(12) **EUROPEAN PATENT APPLICATION**
published in accordance with Art. 153(4) EPC

(43) Date of publication:
**05.01.2022 Bulletin 2022/01**

(51) Int Cl.:
*G06F 3/01* (2006.01)   *G06K 9/00* (2006.01)
*G06N 3/04* (2006.01)

(21) Application number: 20860375.3

(22) Date of filing: 01.09.2020

(86) International application number:
**PCT/CN2020/112766**

(87) International publication number:
**WO 2021/043118 (11.03.2021 Gazette 2021/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 06.09.2019 CN 201910843985

(71) Applicant: TENCENT TECHNOLOGY (SHENZHEN) COMPANY LIMITED
Shenzhen, GUANGDONG 518057, (CN)

(72) Inventors:
• LEI, Mengying
  Shenzhen, Guangdong 518057, (CN)
• DENG, Zijun
  Shenzhen, Guangdong 518057, (CN)
• ZHAO, He
  Shenzhen, Guangdong 518057, (CN)
• ZHENG, Qingqing
  Shenzhen, Guangdong 518057, (CN)
• MA, Kai
  Shenzhen, Guangdong 518057, (CN)
• ZHENG, Yefeng
  Shenzhen, Guangdong 518057, (CN)

(74) Representative: Eisenführ Speiser
Patentanwälte Rechtsanwälte PartGmbB
Postfach 31 02 60
80102 München (DE)

(54) **MOTOR IMAGERY ELECTROENCEPHALOGRAM SIGNAL PROCESSING METHOD, DEVICE, AND STORAGE MEDIUM**

(57)    A motor imagery electroencephalogram signal processing method, a device, and a storage medium. The method comprises: inputting a source MI electroencephalogram signal of a source domain and a target MI electroencephalogram signal of a target domain into an initial feature extraction model separately, and obtaining a first source MI feature used to represent the source MI electroencephalogram signal and a first target MI feature used to represent the target MI electroencephalogram signal (S202); inputting the first source MI feature into an initial classification model, and obtaining a first classification result output by the initial classification model, wherein the first classification result is used to represent a desired execution action of the source MI electroencephalogram signal (S204); and if a first known action corresponding to the source MI electroencephalogram signal is inconsistent with the desired execution action of the source MI electroencephalogram signal as represented by the first classification result, or if a similarity level between a feature distribution of the first source MI feature and a feature distribution of the first target MI feature is less than a first pre-determined threshold, adjusting a model parameter of the initial feature extraction model and/or a model parameter of the initial classification model, and obtaining a target feature extraction model and a target classification model (S206).

Input a source MI electroencephalogram signal belonging to a source domain and a target MI electroencephalogram signal belonging to a target domain to an initial feature extraction model, to obtain first source MI features representing the source MI-EEG signal and first target MI features representing the target MI electroencephalogram signal — S202

Input the first source MI features to an initial classification model, to obtain a first classification result outputed by the initial classification model, the first classification result representing an action predicted to be performed of the source MI electroencephalogram signal — S204

Adjust a model parameter of the initial feature extraction model and/or a model parameter of the initial classification model to obtain a target feature extraction model and a target classification model in a case that a first known action corresponding to the source MI electroencephalogram signal is inconsistent with the action predicted to be performed, represented by the first classification result, of the source MI electroencephalogram signal, or a similarity between a feature distribution of the first source MI features and a feature distribution of the first target MI features is less than a first predetermined threshold — S206

FIG. 2

**Description**

This application claims priority to Chinese Patent Application No. 201910843985.7, filed with the China National Intellectual Property Administration, PRC on September 6, 2019, entitled "MOTOR IMAGERY ELECTROENCEPHALOGRAM SIGNAL PROCESSING METHOD, DEVICE, AND STORAGE MEDIUM", which is incorporated herein by reference in its entirety.

**FIELD**

This application relates to the field of artificial intelligence (AI), and in particular to a technology for processing a motor imagery electroencephalogram signal.

**BACKGROUND**

A motor imagery-brain-computer interface (MI-BCI) is a way for man-machine interaction, which is used to control movement of an external device through spontaneous imagery of limb movement. An MI-BCI-based system may be used to help patients with limb disabilities perform rehabilitation training, and control a machine to achieve self-care to improve living quality, and the MI-BCI-based system may also be used to enrich the life of general populations, for example, a brain-computer game.

At present, because MI electroencephalogram signals vary greatly among individuals, and models of different subjects cannot be shared, it is required to train one independent model based on an electroencephalogram signal of each subject in the MI-BCI system, and then the electroencephalogram signal of the subject may be recognized by using the model obtained through training.

With the above manner, performance of the MI-BCI system is greatly affected by the accuracy of decoding the MI electroencephalogram signal. In addition, the MI-BCI system cannot be applied to untrained subjects, and therefore training should be performed for each subject before actually using the MI-BCI system. Because the training process is cumbersome and complex and there are few training samples of a single subject, the performance of the system is further degraded.

Therefore, there is a problem of a low accuracy of decoding MI electroencephalogram signals of different subjects by an MI recognition model due to the MI electroencephalogram signals varying greatly among subjects in the conventional art.

**SUMMARY**

A method and apparatus for processing an MI electroencephalogram signal and a storage medium are provided according to embodiments of this application, to solve at least the technical problem of a low accuracy of decoding MI electroencephalogram signals of different subjects by an MI recognition model due to the MI electroencephalogram signals varying greatly among subjects in the conventional art.

According to an aspect of the embodiments of this application, a method for processing a motor imagery electroencephalogram signal is provided, including:

inputting a source MI electroencephalogram signal belonging to a source domain and a target MI electroencephalogram signal belonging to a target domain to an initial feature extraction model, to obtain first source MI features representing the source MI electroencephalogram signal and first target MI features representing the target MI electroencephalogram signal;

inputting the first source MI features to an initial classification model, to obtain a first classification result outputted by the initial classification model, the first classification result representing an action predicted to be performed of the source MI electroencephalogram signal; and

adjusting a model parameter of the initial feature extraction model and/or a model parameter of the initial classification model to obtain a target feature extraction model and a target classification model, in a case that a first known action corresponding to the source MI electroencephalogram signal is inconsistent with the action predicted to be performed, represented by the first classification result, of the source MI electroencephalogram signal, or a similarity between a feature distribution of the first source MI features and a feature distribution of the first target MI features is less than a first predetermined threshold, where

a similarity between a feature distribution of second source MI features representing the source MI electroencephalogram signal that are outputted by the target feature extraction model and a feature distribution of second target MI features representing the target MI electroencephalogram signal that are outputted by the target feature extraction model is greater than or equal to the first predetermined threshold, and an action predicted to be performed, represented by a second classification result outputted by the target classification model, of the source MI electroencephalogram signal is consistent with the first known action.

According to another aspect of the embodiments of this application, a method for processing an MI electroencephalogram signal is provided, including:

acquiring a to-be-recognized motor imagery (MI) electroencephalogram signal, the to-be-recognized MI electroencephalogram signal being collected from a target object;

inputting the to-be-recognized MI electroencephalogram signal to a target feature extraction model, to obtain to-be-recognized MI features representing the to-be-recognized MI-EGG signal, and inputting the to-be-recognized MI features to a target classification model, to obtain a target classification result outputted by the target classification model, where

a similarity between feature distributions of different MI features and the to-be-recognized MI features extracted by the target feature extraction model for different MI training signals is greater than or equal to a first similarity threshold,

the different MI training signals includes a target MI electroencephalogram signal and a source MI electroencephalogram signal corresponding to a first known action,

an action predicted to be performed, represented by a classification result outputted by the target classification model, of the source MI electroencephalogram signal is consistent with the first known action,

the classification result is determined by the target classification model based on source MI features extracted from the source MI electroencephalogram signal by the target feature extraction model, and the target classification result represents an action predicted to be performed of the to-be-recognized MI electroencephalogram signal; and

controlling, based on the target classification result, a target device matching the target object to perform the action predicted to be performed of the to-be-recognized MI electroencephalogram signal.

According to still another aspect of the embodiments of this application, an apparatus for processing an MI electroencephalogram signal is further provided, including: a first input unit, a second input unit, and an adjustment unit. The first input unit is configured to input a source MI electroencephalogram signal belonging to a source domain and a target MI electroencephalogram signal belonging to a target domain to an initial feature extraction model, to obtain first source MI features representing the source MI electroencephalogram signal and first target MI features representing the target MI electroencephalogram signal. The second input unit is configured to input the first source MI features to an initial classification model, to obtain a first classification result outputted by the initial classification model, the first classification result represents an action predicted to be performed of the source MI electroencephalogram signal. The adjustment unit is configured to adjust a model parameter of the initial feature extraction model and/or a model parameter of the initial classification model to obtain a target feature extraction model and a target classification model in a case that a first known action corresponding to the source MI electroencephalogram signal is inconsistent with the action predicted to be performed, represented by the first classification result, of the source MI electroencephalogram signal, or a similarity between a feature distribution of the first source MI features and a feature distribution of the first target MI features is less than a first predetermined threshold. A similarity between a feature distribution of second source MI features representing the source MI electroencephalogram signal that are outputted by the target feature extraction model and a feature distribution of second target MI features representing the target MI electroencephalogram signal that are outputted by the target feature extraction model is greater than or equal to the first predetermined threshold, and an action predicted to be performed, represented by a second classification result outputted by the target classification model, of the source MI electroencephalogram signal is consistent with the first known action.

According to still another aspect of the embodiments of this application, an apparatus for processing an MI electroencephalogram signal is further provided, including: a second acquisition unit, a fourth input unit, and a control unit. The second acquisition unit is configured to acquire a to-be-recognized MI electroencephalogram signal, the to-be-recognized MI electroencephalogram signal is collected from a target object. The fourth input unit is configured to input the to-be-recognized MI electroencephalogram signal to a target feature extraction model, to obtain to-be-recognized MI features representing the to-be-recognized MI-EGG signal, and output the to-be-recognized MI features to a target classification model, to obtain a target classification result inputted by the target classification model. A similarity between different MI features extracted by the target feature extraction model for different MI training signals is greater than or equal to a first similarity threshold, the different MI training signals includes a target MI electroencephalogram signal and a source MI electroencephalogram signal corresponding to a first known action, an action predicted to be performed, represented by a classification result outputted by the target classification model, of the source MI electroencephalogram signal is consistent with the first known action, the classification result is determined by the target classification model based on source MI features extracted from the source MI electroencephalogram signal by the target feature extraction model, and the target classification result represents an action predicted to be performed of the to-be-recognized MI electroencephalogram signal. The control unit is configured to control, based on the target classification result, a target device matching the target object to perform the action predicted to be performed of the to-be-recognized MI electroencephalogram signal.

According to still another aspect of the embodiments of this application, a computer-readable storage medium is further provided, storing a computer program. The computer program, when run, performs the above method.

According to still another aspect of the embodiments of this application, an electronic device is further provided, including a memory, a processor, and a computer program stored in the memory and executable on the processor. The above processor, when executing the computer program, performs the above method.

According to still another aspect of the embodiments of this application, a computer program product is further provided. the computer program product, when run on a computer, causes the computer to perform the above method.

In the embodiments of this application, the feature extraction model and the classification model are trained by using the source MI electroencephalogram signal corresponding to the first known action and the target MI electroencephalogram signal, so that the features of the source MI electroencephalogram signal and the features of the target MI electroencephalogram signal that are extracted by the feature extraction model are distributed similarly, and the classification model can correctly recognize the first known action. Because the features of the target MI electroencephalogram signal and the features of the source MI-EGG signal are distributed similarly, the target feature extraction model and the target classification model obtained may be applied to recognize the MI electroencephalogram signals of the target object, so as to improve the generalization capability of the MI recognition model (including the feature extraction model and the classification model obtained) and the accuracy of decoding (classification), thereby solving the technical problem of a low accuracy of decoding MI electroencephalogram signals of different subjects by an MI recognition model due to the MI electroencephalogram signals varying greatly among subjects in the conventional art.

## BRIEF DESCRIPTION OF THE DRAWINGS

FIG. 1 is a schematic diagram of an application environment of a method for processing an MI electroencephalogram signal according to an embodiment of this application.

FIG. 2 is a schematic flowchart of a method for processing an MI electroencephalogram signal according to an embodiment of this application.

FIG. 3 is a schematic diagram of a method for processing an MI electroencephalogram signal according to an embodiment of this application.

FIG. 4 is a schematic diagram of a method for processing an MI electroencephalogram signal according to another embodiment of this application.

FIG. 5 is a schematic diagram of a method for processing an MI electroencephalogram signal according to another embodiment of this application.

FIG. 6 is a schematic diagram of a method for processing an MI electroencephalogram signal according to another embodiment of this application.

FIG. 7 is a schematic flowchart of a method for processing an MI electroencephalogram signal according to another embodiment of this application.

FIG. 8 is a schematic flowchart of a method for processing an MI electroencephalogram signal according to another embodiment of this application.

FIG. 9 is a schematic flowchart of another method for processing an MI electroencephalogram signal according to an embodiment of this application.

FIG. 10 is a schematic structural diagram of an apparatus for processing an MI electroencephalogram signal according to an embodiment of this application.

FIG. 11 is a schematic structural diagram of another apparatus for processing an MI electroencephalogram signal according to an embodiment of this application.

FIG. 12 is a schematic structural diagram of an electronic device according to an embodiment of this application.

**DESCRIPTION OF EMBODIMENTS**

Technical terms involved in embodiments of this application are described as follows.

(1) A BCI refers to a mode of a channel for exchanging information between a brain of a human or an animal and an external machine.

(2) An electroencephalogram (EEG) refers to a curve graph obtained by collecting biological voltages of the brain in the scalp by using a non-invasive brain-computer interface device and magnifying for recording.

(3) MI refers to imagining limb movement in mind without any limb movement, which creates spontaneous electroencephalogram.

(4) A convolutional neural network (CNN) is a feedforward neural network that contains convolution calculation and has a deep structure, which is one of representative algorithms of deep learning.

(5) Transfer learning refers to storing a solution model of existing problems, and applying the model to other related but different problems.

(6) Domain adaptation is intended to cause a source domain to be generalized to a target domain with different data distributions by using a model obtained through supervised learning. Data of the source domain and the target domain is mapped to one feature space, so that the data is distributed close to each other in the space as far as possible, thereby improving the processing performance of the model in the target domain.

(7) A generative adversarial network (GAN) is used to cause, through unsupervised learning, a generator and a discriminator of two neural networks to contest with each other for learning.

(8) A maximum mean discrepancy (discrepancy measure) is used to measure a similarity between two distributions.

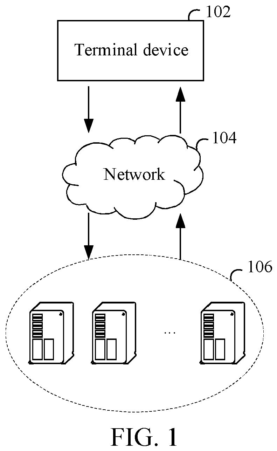

According to an aspect of the embodiments of this application, a method for processing a motor imagery electroencephalogram signal is provided. Exemplarily, the method for processing an motor imagery electroencephalogram signal may be applied to, but is not limited to, an application environment shown in FIG. 1. As shown in FIG. 1, the method for processing an motor imagery electroencephalogram signal involves interaction between a terminal device 102 and a server 106 by using a network 104.

The terminal device 102 may collect a source MI electroencephalogram signal and a target MI electroencephalogram signal or acquire the source MI electroencephalogram signal and the target MI electroencephalogram signal from other devices, and transmit the acquired source MI electroencephalogram signal and target MI electroencephalogram signal to the server 106 through the network 104. The source MI electroencephalogram signal and the target MI electroencephalogram signal may belong to different objects (subjects), and the source MI electroencephalogram signal corresponds to a first known action. The source MI electroencephalogram signal belongs to a source domain, and the target MI electroencephalogram signal belongs to a target domain. The source domain includes the MI electroencephalogram signal corresponding to a determined known action, and the target domain may include the MI electroencephalogram signal corresponding to an undetermined known action.

After the server 106 acquires the above source MI electroencephalogram signal and the target MI electroencephalogram signal, the source MI electroencephalogram signal and the target MI electroencephalogram signal may be inputted to an initial feature extraction model, to obtain first source MI features representing the source MI electroencephalogram signal and first target MI features representing the target MI electroencephalogram signal. The first source MI features are inputted to an initial classification model, to obtain a first classification result outputted by the initial classification model. The first classification result represents an action predicted to be performed of the source MI electroencephalogram signal. In a case that a first known action corresponding to the source MI electroencephalogram signal is inconsistent with the action predicted to be performed, represented by the first classification result, of the source MI electroencephalogram signal, or a similarity between a feature distribution of the first source MI features and a feature distribution of the first target MI features is less than a first predetermined threshold, a model parameter of the initial feature extraction model and/or a model parameter of the initial classification model are/is adjusted to obtain a target feature extraction model and a target classification model. A similarity between a feature distribution of second source MI features representing the source MI electroencephalogram signal that are outputted by the target feature extraction model and a feature distribution of second target MI features representing the target MI electroencephalogram signal that are outputted by the target feature extraction model is greater than or equal to the first predetermined threshold, and an action predicted to be performed, represented by a second classification result outputted by the target classification model, of the source MI electroencephalogram signal is consistent with the first known action corresponding to the source MI electroencephalogram signal.

Exemplarily, the target feature extraction model and the target classification model may serve as a feature extractor and a classifier of an MI recognition model, and may be used to test the MI recognition model by using a test sample in a test set, to determine the performance of the model, or may be used to acquire a to-be-recognized MI electroencephalogram signal, recognize an action predicted to be performed of the to-be-recognized MI electroencephalogram signal, and control a device to perform the action predicted to be performed of the to-be-recognized MI electroencephalogram signal.

Exemplarily, in this embodiment, the above terminal device may include, but is not limited to, a signal collection device configured to collect the MI electroencephalogram signal. The above network may include, but is not limited to, at least one of a wireless network and a wired network. The wireless network includes: Bluetooth, Wi-Fi, or another network implementing wireless communication, and the wired network may include: a local area network, a metropolitan area network, or a wide area network. The above server may include, but is not limited to, a server configured to train the MI recognition model. The foregoing description is merely an example, and is not limited in this embodiment.

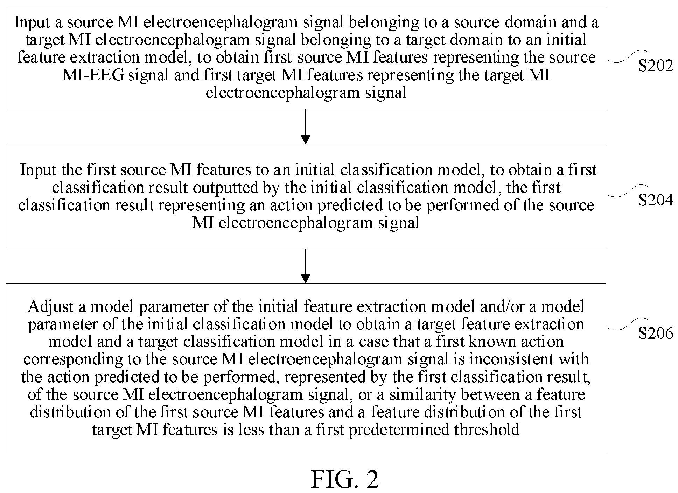

Exemplarily, in this embodiment, as an exemplary implementation, as shown in FIG. 2, the method for processing an MI electroencephalogram signal may include the following steps S202 to 206.

In step S202, a source MI electroencephalogram signal belonging to a source domain and a target MI electroencephalogram signal belonging to a target domain are inputted to an initial feature extraction model, to obtain first source MI features representing the source MI electroencephalogram signal and first target MI features representing the target MI electroencephalogram signal.

In step S204, the first source MI features are inputted to an initial classification model, to obtain a first classification result outputted by the initial classification model. The first classification result represents an action predicted to be performed of the source MI electroencephalogram signal.

In step S206, a model parameter of the initial feature extraction model and/or a model parameter of the initial classification model are adjusted to obtain a target feature extraction model and a target classification model in a case that a first known action corresponding to the source MI electroencephalogram signal is inconsistent with the action predicted to be performed, represented by the first classification result, of the source MI electroencephalogram signal, or a similarity between a feature distribution of the first source MI features and a feature distribution of the first target MI features is less than a first predetermined threshold. A similarity between a feature distribution of second source MI features representing the source MI electroencephalogram signal that are outputted by the target feature extraction model and a feature distribution of second target MI features representing the target MI electroencephalogram signal that are outputted by the target feature extraction model is greater than or equal to the first predetermined threshold, and an action predicted to be performed, represented by a second classification result outputted by the target classification model, of the source MI electroencephalogram signal is consistent with the first known action.

Exemplarily, the target feature extraction model and the target classification model obtained by the method for processing an MI electroencephalogram signal may serve as an MI recognition model. The MI recognition model may be applied to, but is not limited to, the processes of transmission and control of ideas. For example, a BCI system combined with an exoskeleton robot may be used for active rehabilitation of motor functions of patients with hemiplegia or cerebral stroke, a BCI system combined with an electric wheelchair may help users with physical disabilities to travel freely, and a brain-computer-game system combined with a game may achieve activities of objects (including, but not limited to, at least one of the following: characters, articles, and the like) in a virtual world controlled by a human body through ideas and imagination.

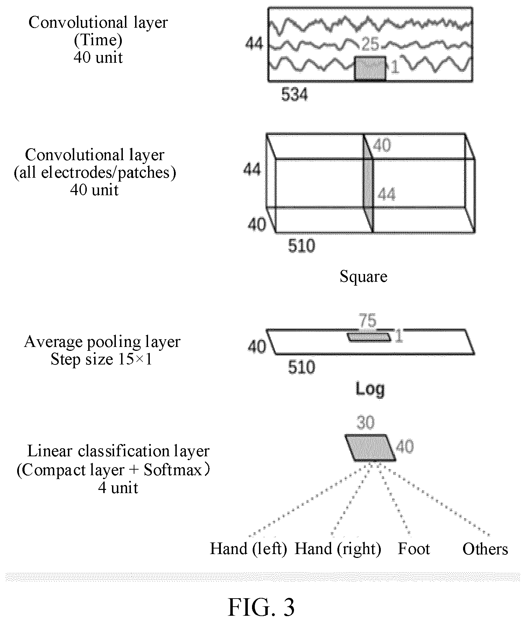

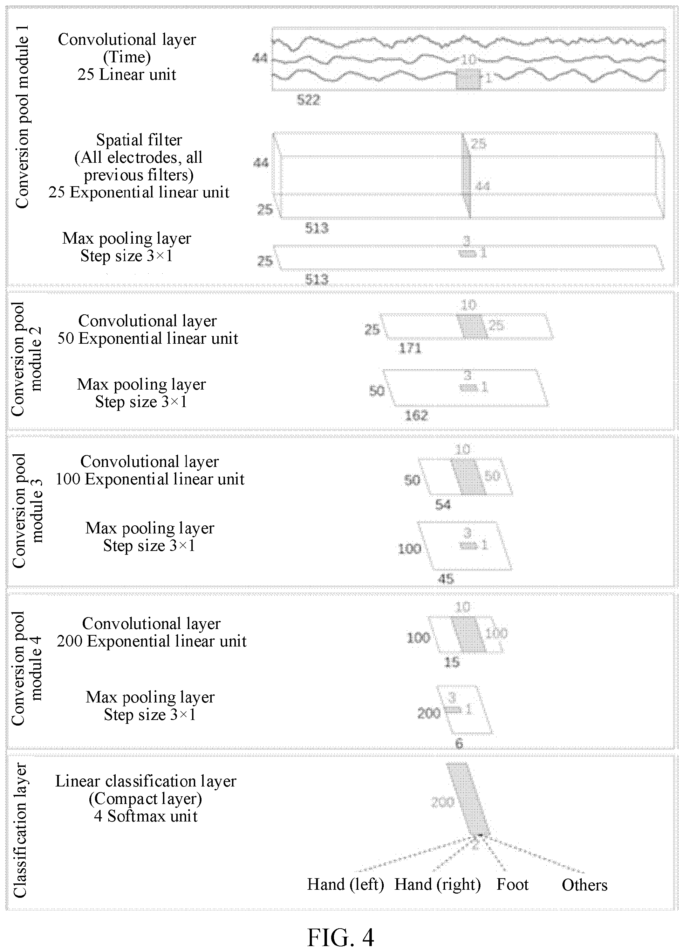

The MI recognition manners in the conventional art may include a manner of an MI electroencephalogram classification algorithm based on a shallow convolutional network (Shallow ConvNet) and a manner of an MI electroencephalogram classification algorithm based on a deep convolutional network (Deep ConvNet). As shown in FIG. 3, an original electroencephalogram signal is used as a network input of the MI classification of the Shallow ConvNet, taking advantages of high temporal resolution and rich spatial information of the electroencephalogram signal, by using a spatiotemporal way of extracting the electroencephalogram signal through one-dimensional time and space convolution, and through average pooling, full connectivity, and softmax, a classification probability corresponding to the signal is obtained. As shown in FIG. 4, a deep network structure of the Deep ConvNet is based on a shallow spatiotemporal convolution structure, and multiple convolutional pooling layers are added in the time dimension, to make use of electroencephalogram time information.

Since the EEG classification algorithm based on the Shallow ConvNet involves a simple model structure, extracted feature information is limited and is not enough to fit complex MI electroencephalogram signals. The deep network based on the Deep ConvNet is formed by directly expanding the Shallow ConvNet by increasing quantities of convolutional layers and pooling layers, and the training process requires more training samples, otherwise severe overfitting may be caused, which may result in poor generalization capability of the model.

Although the EEG classification method based on the Shallow ConvNet and the EEG classification method based on the Deep ConvNet both achieve end-to-end automatic recognition and decoding on electroencephalogram signals, due to large differences in signal of different subjects, the algorithms of the two network structures may only be trained independently for the training samples of each of different subjects, so that the models trained for different subjects cannot learn from each other, resulting in poor general transference of the models. In addition, the two algorithms both are supervised learning methods and cannot be used to train and recognize the MI electroencephalogram signals of unlabeled target subjects.

With this embodiment, the feature extraction model and the classification model are trained by using the source MI electroencephalogram signal corresponding to the first known action and the target MI electroencephalogram signal, so that the features of the source MI electroencephalogram signal and the features of the target MI electroencephalogram signal that are extracted by the feature extraction model are distributed similarly, and the classification model can correctly recognize the first known action, which mitigates the technical problem of a low accuracy of decoding MI electroencephalogram signals of different subjects by an MI recognition model due to the MI electroencephalogram signals varying greatly among subjects in the conventional art, and improves the generalization capability of the MI recognition model (including the obtained feature extraction model and classification model) and the accuracy of decoding.

The method for processing an MI electroencephalogram signal is described below with reference to FIG. 2.

In step S202, the source MI electroencephalogram signal belonging to the source domain and the target MI electroencephalogram signal belonging to the target domain are inputted to the initial feature extraction model, to obtain the first source MI features representing the source MI electroencephalogram signal and the first target MI features representing the target MI electroencephalogram signal.

The MI recognition model used to recognize the MI electroencephalogram signal may include a feature extraction model (a feature extractor) and a classification model (a classifier). In order to obtain the MI recognition model, subject training data (training data of subjects) may be used for model training. Training the MI recognition model may include a process of adjusting a model parameter of an initial feature extraction model and/or a model parameter of an initial classification model.

The training data of different subjects may be distributed to the source domain and the target domain. For example, an electroencephalogram signal of a target subject is assigned to the target domain, and the electroencephalogram signals of other subjects with high decoding performance are assigned to the source domain. The target MI electroencephalogram signal in the target domain may or may not have a classification label. The source MI electroencephalogram signal in the source domain has a classification label, the classification label may be used to identify the first known action corresponding to the source MI electroencephalogram signal, and the first known action may be one of multiple known actions.

For example, in the training process, MI-EEG samples of different subjects may be distributed to the source domain and the target domain. The distribution may be performed according to specific cases. If there is data with no label in a subject data set, subject data with labels may be assigned to the source domain, and subject data with no label may be assigned to the target domain. If all subject data has labels, subject data of any one of subjects may be assigned to the target domain, and subject data of another subject is assigned to the source domain, which may be repeated until subject data of all the subjects has been assigned to the target domain.

Multiple known actions may be set according to experience, which includes, but is not limited to, at least one of the following: a left-hand action (for example, clenching a left hand), a right-hand action (for example, clenching a right hand), a tongue action, a two-foot action (a left-foot action and a right-foot action may or may not be distinguished), and the like. Different classification labels may correspond to one of multiple known actions, and the same MI electroencephalogram signal may correspond to one known action. Different MI electroencephalogram signals may correspond to the same known action or may correspond to different known actions.

Exemplarily, in this embodiment, before the source MI electroencephalogram signal belonging to the source domain and the target MI electroencephalogram signal belonging to the target domain are inputted to the initial feature extraction model, the server may transmit a target instruction to the collection device, where the target instruction indicates the first known action to be performed, and receive the source MI electroencephalogram signal transmitted from the collection device, where the source MI electroencephalogram signal is collected by the collection device within a preset time period after receiving the target instruction.

In order to acquire the source MI electroencephalogram signal, the server may transmit the target instruction to the collection device (an MI electroencephalogram signal collection device), and the target instruction indicates the first known action to be performed.

According to relevant protocol standards, an electroencephalogram signal of an object (for example, a human) may be collected at a predetermined location. There may be multiple (for example, 60) predetermined locations, which are distributed across a human head, and each location corresponds to a channel. The collection device may be connected to multiple patches to collect electroencephalogram signals, where different patches are used to collect electroencephalogram signals of different channels.

The patches on the collection device may correspond to all or part of locations in the relevant protocol. The locations of the patches for collecting the electroencephalogram signals and the number of the patches may be set as required, which is not limited in this embodiment.

For example, the protocol stipulates that there are N locations on the human head at which electroencephalogram signals are collected, which correspond to N different channels. Electroencephalogram signals of all the N channels or a preset number of channels may be collected, and electroencephalogram signals of all the channels or only electroencephalogram signals of specific channels may be used during the processing.

After receiving the target instruction, the collection device may prompt the subject using the collection device with the first known action, and prompt the subject to imagine the first known action in mind, so as to collect the electroencephalogram signal of the subject, to obtain the source MI electroencephalogram signal. The collection device may collect the MI electroencephalogram signals (including the electroencephalogram signals of different channels) within a preset time period after receiving the target instruction.

The preset time period may be a time period during which the subject is in a motor imagery state after a first time at which the target instruction is received, for example, during a time period from the first second to the 5.5th second after the first time. Alternatively, the preset time period may be a time period during which the subject is in an MI state after a second time at which a prompt message (for prompting the subject to imagine the first known action) is sent after the target instruction is received, for example, during a time period from the first second to the 5.5th second after the second time.

With this embodiment, the collection device is instructed to collect MI electroencephalogram signals in response to the target instruction indicating the to-be-performed first known action, so that the source MI electroencephalogram signal corresponding to the first known action can be obtained, thereby ensuring the accuracy of acquiring the source MI electroencephalogram signal.

The target MI electroencephalogram signal may be acquired in the same way as the source MI electroencephalogram signal (the target MI electroencephalogram signal corresponding to a second known action), or may be acquired in a different way. For example, the target instruction does not indicate a specific known action, and the subject spontaneously imagines one of multiple known actions, to acquire the target MI electroencephalogram signal.

Exemplarily, in this embodiment, in order to improve the efficiency of acquiring training samples (the source MI electroencephalogram signal and the target MI electroencephalogram signal), a public data set may be used as training data. The labeled motor imagery training data set may be used as the source domain, the unlabeled motor imagery training data set may be used as the target domain, and MI-EEG signals of on-line unknown users may be used as the test set. The above public data set may be public competition data, for example, public motor imagery data for the 3rd China BCI competition.

Exemplarily, in this embodiment, before the source MI electroencephalogram signal belonging to the source domain and the target MI electroencephalogram signal belonging to the target domain are inputted to the initial feature extraction model, an initial source MI electroencephalogram signal belonging to the source domain and an initial target MI electroencephalogram signal belonging to the target domain may be acquired; the initial source MI electroencephalogram signal is preprocessed to obtain the source MI electroencephalogram signal; and the initial target MI electroencephalogram signal is preprocessed to obtain the target MI electroencephalogram signal.

In the above way, the source MI electroencephalogram signal and the target MI electroencephalogram signal may be directly acquired, or the initial source MI electroencephalogram signal and the initial target MI electroencephalogram signal may be acquired, and the initial source MI electroencephalogram signal and the initial target MI electroencephalogram signal are preprocessed to obtain the source MI electroencephalogram signal and the target MI electroencephalogram signal. The preprocessing of the MI electroencephalogram signal may include standardizing the MI electroencephalogram signal, so that the preprocessed MI electroencephalogram signal may be used for training an MI electroencephalogram signal recognition model.

In this embodiment, the initial source MI electroencephalogram signal and the initial target MI electroencephalogram signal are preprocessed, so that the MI electroencephalogram signal can be optimized, thereby improving the effectiveness of training the MI electroencephalogram signal recognition model.

In an exemplary implementation, the preprocessing the initial source MI electroencephalogram signal to obtain the source MI electroencephalogram signal includes: intercepting a signal of a predetermined duration from the initial source MI electroencephalogram signal, to serve as a first source MI electroencephalogram signal; inputting the first source MI electroencephalogram signal to a band-pass filter, to obtain a second source MI electroencephalogram signal, where the band-pass filter is configured to filter out, from the first source MI electroencephalogram signal, a signal not in a band-pass frequency band range; and standardizing the second source MI electroencephalogram signal to obtain the source MI electroencephalogram signal.

In another exemplary implementation, the preprocessing the initial target MI electroencephalogram signal to obtain the target MI electroencephalogram signal includes: intercepting a signal of a predetermined duration from the initial target MI electroencephalogram signal, to serve as a first target MI electroencephalogram signal; inputting the first target MI electroencephalogram signal to a band-pass filter, to obtain a second target MI electroencephalogram signal, where the band-pass filter is configured to filter out, from the first target MI electroencephalogram signal, a signal not in a band-pass frequency band range; and standardizing the second target MI electroencephalogram signal to obtain the target MI electroencephalogram signal.

The preprocessing of the MI electroencephalogram signal (which may include the initial source MI electroencephalogram signal and/or the initial target MI electroencephalogram signal) may include, but is not limited to, at least one of the following: duration normalization, selection of a channel, denoising (low-pass filtering or band-pass filtering), and signal standardization.

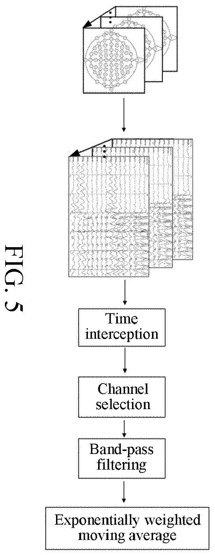

The preprocessing process of MI electroencephalogram signals is described below with examples. As shown in FIG. 5, the preprocessing operation on the MI electroencephalogram signal may include, but is not limited to, at least one of the following operations.

1) Intercepting time period signals related to motor imagery (duration normalization).

Signals within an motor imagery interval are intercepted for each sample, and data for a total of 4.5s from the first second to the 5.5th second may be intercepted. Because a signal sampling frequency is 1000 Hz, a time dimension of each sample is 4500.

2) Channel selection

Channel signals that are not related to MI tasks may be removed via channel selection. The selected channels may include 20 channels related to MI tasks (including FCz, FC1, FC2, FC3, FC4, FC5, FC6, Cz, C1, C2, C3, C4, C5, C6, CP1, CP2, CP3, CP4, CP5, and CP6), and the selected channels may also include more, less, or different channels.

3) Filtering

The noise caused due to blinking, an environmental noise, and equipment, and the like may be removed by a band-pass filtering process. The band-pass filter may be a third-order Butterworth filter, and has a band-pass range of 4Hz to 38 Hz or of 7Hz to 30 Hz. Since useful information in the MI electroencephalogram signal is mainly concentrated in a low-frequency region, the useful information in the MI electroencephalogram signal may be retained while filtering out the noise by using the band-pass filter.

4) Exponentially weighted moving average

For signals obtained by smoothing filtering (signals obtained by band-pass filtering), in order to further reduce signal fluctuation caused by noise, the exponentially weighted moving average operation may be used for standardizing the signal. The signal standardization is realized with the exponentially weighted moving average method, and a weight parameter may be set to 0.999. Other standardization operations may be used, such as mean variance standardization, or a CSP algorithm.

With this embodiment, the initial MI electroencephalogram signal is preprocessed to filter out noise data, and lengths of the MI electroencephalogram signals are unified and the MI electroencephalogram signals are standardized, thereby improving the effectiveness of training the MI electroencephalogram signal recognition model.

The source MI electroencephalogram signal and the target MI electroencephalogram signal may be inputted to the initial feature extraction model after being obtained, to obtain the first source MI features representing the source MI electroencephalogram signal and the first target MI features representing the target MI electroencephalogram signal.

The above initial feature extraction model may be a feature extractor that processes electroencephalogram signals based on the CNN, or may be a feature extractor having another network structure (such as ResNet and LSTM).

In step S204, the first source MI features are inputted to the initial classification model, to obtain the first classification result outputted by the initial classification model, the first classification result represents the action predicted to be performed of the source MI electroencephalogram signal.

The first source MI features extracted by the initial feature extraction model may be inputted to the initial classification model to obtain the first classification result outputted by the initial classification model that represents the action predicted to be performed of the source MI electroencephalogram signal. The initial classification model may be a classifier that processes electroencephalogram signals based on the CNN, and the classification result may indicate an action (corresponding to a known action having the highest probability) predicted to be performed of the source MI electroencephalogram signal, the action indicated by the classification result is determined based on probabilities of known actions predicted to be performed of the source MI electroencephalogram signal that are determined by the first source MI features.

The feature extraction model and the classification model may be different submodels of the MI electroencephalogram signal recognition model. The structures of the feature extraction model and the classification model may be pre-configured. The model parameter of the initial feature extraction model and the model parameter of the initial classification model may be randomly initialized (for example, initialized to 0), or may be obtained by training a randomly initialized initial feature extraction model and a randomly initialized initial classification model by using the source MI electroencephalogram signal.

For example, for the spatiotemporal attribute of the electroencephalogram signal, the feature extractor may adopt a temporal convolutional layer and a spatial convolutional layer. Based on the Deep ConvNet, the model parameters of the feature extractor and the classifier may be as shown in Table 1. The network structure may be determined by modifying the number of convolutional pooling layers and using convolution kernels of different sizes.

Table 1

| Layer name | Output size | Network |
|---|---|---|
| Output layer | $20 \times 4500$ | -- |
| Temporal convolutional layer | $20 \times 4491$ | $1 \times 10$, 25, stride $1 \times 1$ |
| Spatial convolutional layer | $1 \times 4491$ | $20 \times 1$, 25, stride $1 \times 1$ |
| Max pooling layer | $1 \times 1497$ | $1 \times 3$, stride $1 \times 3$ |
| Convolutional layer 2 | $1 \times 1488$ | $1 \times 10$, 50, stride $1 \times 1$ |
| Max pooling layer | $1 \times 496$ | $1 \times 3$, stride $1 \times 3$ |
| Convolutional layer 3 | $1 \times 487$ | $1 \times 10$, 100, stride $1 \times 1$ |
| Max pooling layer | $1 \times 162$ | $1 \times 3$, stride $1 \times 3$ |
| Convolutional layer 4 | $1 \times 153$ | $1 \times 10$, 200, stride $1 \times 1$ |
| Max pooling layer | $1 \times 51$ | $1 \times 3$, stride $1 \times 3$ |
| Flattening layer | 10200 | -- |
| Fully connected layer | 1024 | 1024 |
| Fully connected layer | 1024 | 1024 |
| Output layer | 2 | 2 |

Exemplarily, in a case that the target MI electroencephalogram signal corresponds to the second known action, in this embodiment, the first target MI features extracted by the initial feature extraction model for the target MI electroencephalogram signal may further be inputted to the initial classification model, to obtain a third classification result outputted by the initial classification model. The third classification result represents an action predicted to be performed of the target MI electroencephalogram signal. It is to be noted that the third classification result may indicate an action (corresponding to a known action having the highest probability) predicted to be performed of the target MI electroencephalogram signal, the action indicated by the third classification result is determined based on probabilities of known actions predicted to be performed of the target MI electroencephalogram signal that are determined by the first target MI features.

In step S206, the model parameter of the initial feature extraction model and/or the model parameter of the initial classification model are/is adjusted to obtain the target feature extraction model and the target classification model, in a case that the first known action corresponding to the source MI electroencephalogram signal is inconsistent with the action predicted to be performed, represented by the first classification result, of the source MI electroencephalogram signal, or a similarity between the feature distribution of the first source MI features and the feature distribution of the first target MI features is less than the first predetermined threshold.

That is, after the first source MI features, the first target MI features, and the first classification result are obtained, it may be determined whether the first known action corresponding to the source MI electroencephalogram signal is consistent with the action predicted to be performed, represented by the first classification result, of the source MI electroencephalogram signal, and whether a similarity between the feature distribution of the first source MI features and the feature distribution of the first target MI features is less than the first predetermined threshold. The first predetermined threshold may be a preset fixed value, or may be a value determined based on an objective function.

In a case that a first known action corresponding to the source MI electroencephalogram signal is inconsistent with the action predicted to be performed, represented by the first classification result, of the source MI electroencephalogram signal, or a similarity between a feature distribution of the first source MI features and a feature distribution of the first target MI features is less than a first predetermined threshold, it may be determined that the model does not converge, and the model parameter of the initial feature extraction model and/or the model parameter of the initial classification model need/needs to be adjusted, to obtain a target feature extraction model and a target classification model. A similarity between a feature distribution of second source MI features representing the source MI electroencephalogram signal that are outputted by the target feature extraction model and a feature distribution of second target MI features representing the target MI electroencephalogram signal that are outputted by the target feature extraction model is greater than or equal to the first predetermined threshold, and an action predicted to be performed, represented by a second classification result outputted by the target classification model, of the source MI electroencephalogram signal is consistent with the first known action. The second classification result indicates an action (corresponding to a known action having the highest probability) predicted to be performed of the source MI electroencephalogram signal, the action indicated by the second classification result is determined by the target classification model based on probabilities of known actions predicted to be performed of the source MI electroencephalogram signal that are determined by the second source MI features.

Exemplarily, in a case that the target MI electroencephalogram signal corresponds to the second known action, and the third classification result representing the action predicted to be performed of the target MI electroencephalogram signal has been determined by using the initial classification model, in step 206 of the method in the embodiments of this application, the step of adjusting the model parameter of the initial feature extraction model and/or the model parameter of the initial classification model to obtain the target feature extraction model and the target classification model may be performed in a case that the first known action is inconsistent with the action predicted to be performed, represented by the first classification result, of the source MI electroencephalogram signal, or that the second known action is inconsistent with the action predicted to be performed, represented by the third classification result, of the target MI electroencephalogram signal, or that a similarity between the feature distribution of the first source MI features and the feature distribution of the first target MI features is less than the first predetermined threshold.

For example, the similarity between the feature distribution of the first source MI features and the feature distribution of the first target MI features may be determined, for example, by using a domain discrimination model, or according to another metric such as a cosine distance, a Manhattan distance, and a Chebyshev distance.

In an exemplary implementation, the adjusting a model parameter of the initial feature extraction model and/or a model parameter of the initial classification model to obtain a target feature extraction model and a target classification model includes: inputting the first source MI features and the first target MI features to an initial domain discrimination model, to obtain a first source determination result and a first target determination result. The initial domain discrimination model is configured to: determine, based on the feature distribution of the first source MI features, a probability that the source MI electroencephalogram signal belongs to the source domain and a probability that the source MI electroencephalogram signal belongs to the target domain, and determine, based on the feature distribution of the first target MI features, a probability that the target MI electroencephalogram signal belongs to the source domain and a probability that the target MI electroencephalogram signal belongs to the target domain.

Further, the model parameter of the initial MI recognition model (which includes the initial feature extraction model and the initial classification model) and a model parameter of the initial domain discrimination model are successively adjusted by using the source MI electroencephalogram signal and the target MI electroencephalogram signal via multiple iterations of training, to obtain the target MI recognition model (which includes the target feature extraction model and the target classification model) and a target domain discrimination model. The target domain discrimination model may determine, based on the second source MI features (features extracted from the source MI electroencephalogram signal by the target feature extraction model), a probability that the source MI electroencephalogram signal belongs to the source domain and a probability that the source MI electroencephalogram signal belongs to the target domain, where a difference between the two probabilities is less than or equal to a second predetermined threshold, and the second predetermined threshold may be fixed, or may be changed based on a target loss function.

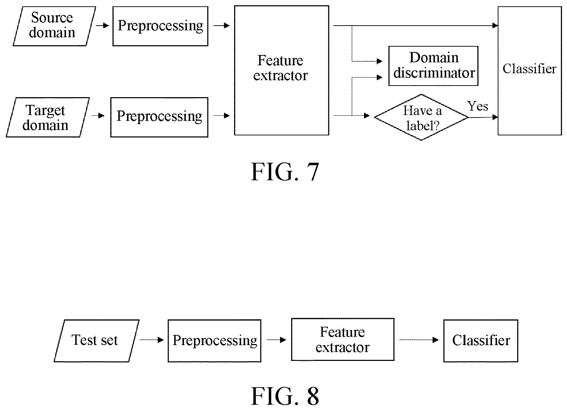

In an implementation, as shown in FIG. 7, preprocessed signals (including the source MI electroencephalogram signal from the source domain and the target MI electroencephalogram signal from the target domain) may be inputted to a feature extractor (the initial feature extraction model) which is designed based on the CNN to process electroencephalogram signals, a classifier (the initial classification model), and a domain discriminator (the initial domain discrimination model). During the training process, after the feature extractor and the classifier are updated, the domain discriminator is updated, and loop iterations are thus performed. A final model classifier (the MI recognition model including the target feature extraction model and the target classification model) can accurately recognize the motor imagery classification included in the inputted electroencephalogram signal. The domain discriminator may have a multi-layer fully connected structure based on the CNN, and the network structure may be as shown in FIG. 2.

Table 2

| Layer name | Output size | Network |
|---|---|---|
| Input layer | 10200 | -- |
| Fully connected layer | 1024 | 1024 |
| Fully connected layer | 512 | 512 |
| Fully connected layer | 1 | 1 |

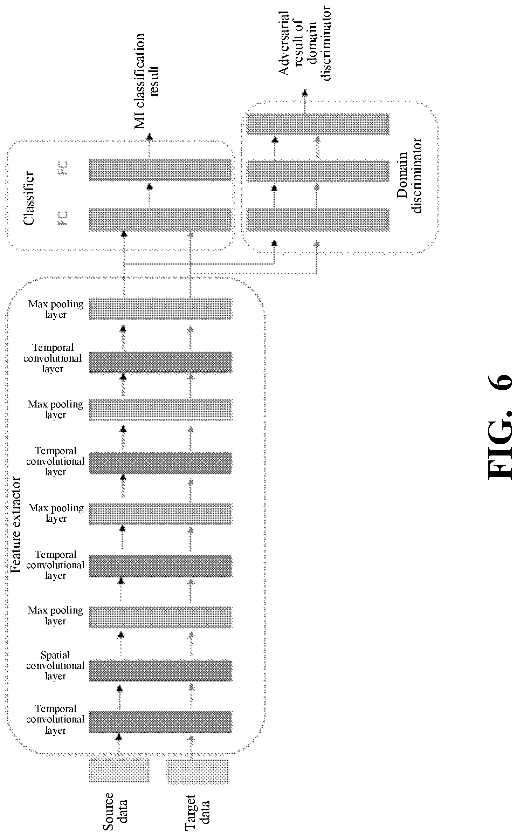

As shown in FIG. 6, a training model including the feature extractor, the classifier, and the domain discriminator may be a domain-adaptive motor imagery deep decoding model based on discrimination adversary. Motor imagery-related features of subjects in the source domain are extracted through deep learning for classification and decoding. In addition, by using the domain-adaptive branch of the discrimination network adversary, deep electroencephalogram signal features of target subjects and the features of the source domain reach an indistinguishable balance.

In order to further optimize the discrimination network branch, "metric learning" may be used, and a maximum mean difference (MMD) loss function and an adversarial loss function are constrained, so as to further reduce distribution distances of the electroencephalogram signal features between the subject in the source domain and the target subject, thereby improving the decoding accuracy on the target subject.

Exemplarily, the loss function (objective function) used for loop iterations may include a loss function $L_G$ for a branch of the feature extractor and classifier, and a loss function $L_D$ for a branch of the a domain discriminator. $L_G$ includes the following three loss functions

1) a loss function $L_{ce}$ used to determine the motor imagery classification, which may be a cross-entropy loss function;
2) an adversarial loss function $L_{Gadv}$; and
3) a loss function $L_{mmd}$ used to determine a similarity of distributions of the features generated by the feature extractor in the source domain and the target domain.

The loss functions may be expressed according to the following equations in (1):

$$\begin{cases} L_G = L_{ce} + \alpha L_{Gadv} + \beta L_{mmd} \\ L_{ce} = -[y \log \hat{y} + (1-y)\log(1-\hat{y})] \\ L_{Gadv} = \log(1 - D(G(z))) \\ L_{mmd} = \frac{1}{c}\sum_{j=1}^{c} \sup_f \left\| \frac{1}{m_j} f(x_i^j) - \frac{1}{n_j} f(z_i^j) \right\|^2 \end{cases}$$

$$(1)$$

where $\alpha$ and $\beta$ represent weights of different loss functions, $y$ represents an actual label, and $\hat{y}$ represents a prediction label. D and G respectively represent a domain discriminator network and a feature extractor network, x represents a sample in the source domain, z represents a sample in the target domain, and C represents the number of categories, f represents feature layers of the feature extractor. $m_j$ represents the number of the j$^{th}$ category of samples in the source

domain, and $n_j$ represents the number of the j$^{th}$ category of samples in the target domain.

The domain discriminator may adopt a minimum adversarial loss function, which may be expressed by the following equation (2):

$$L_{Dadv} = -\log D(G(x)) - \log(1 - D(G(z)))$$

$$(2)$$

With the transfer learning method based on domain discriminant adversarial learning, by using the electroencephalogram data of other subjects, the problem of overfitting caused by a small amount of subject independent training data and a large number of deep learning parameters is solved, and the accuracy of the model of the target subject is also improved. The method for processing an MI electroencephalogram signal is described. With the method for processing an MI electroencephalogram signal in this example, the problem of a small amount of training data for electroencephalogram signals of a single subject can be solved, to greatly improve the application range of a deep convolutional network on small sample electroencephalogram signals, so as to provide more possibilities for decoding electroencephalogram signals by deep models. The method for processing an MI electroencephalogram signal in this example may be performed by a server, and may include a training phase and a test phase.

In the training phase, a source MI electroencephalogram signal belonging to a source domain and a target MI electroencephalogram signal belonging to a target domain are inputted to an initial feature extraction model, to obtain first source MI features and first target MI features; the first source MI features are inputted to an initial classification model, to obtain a first classification result outputted by the initial classification model, where the first classification result represents a prediction of the initial classification model for an action performed by the source MI electroencephalogram signal; and the source MI electroencephalogram features and the target electroencephalogram features are inputted to the domain discrimination model to predict the sources of the inputted electroencephalogram features.

In a case that the first known action corresponding to the source MI electroencephalogram signal is inconsistent with the action performed by the source MI electroencephalogram signal that is predicted by the initial classification model, or a similarity between the feature distribution of the first source MI features and the feature distribution of the first target MI features is less than the first predetermined threshold, the model loss function is minimized based on the inputted first source/target electroencephalogram signal, and the model parameter of the initial feature extraction model, and/or the model parameter of the initial classification model, and/or the model parameter of the domain discrimination model are/is adjusted to obtain the target feature extraction model and the target classification model.

In the test phase, the target MI electroencephalogram signal is inputted to the target extraction model and the target classification model, so that the action performed by the target MI electroencephalogram signal may be predicted. After the MI recognition model is obtained, the target subject test set may be used for verifying the accuracy and generalization capability of the MI recognition model obtained through training or for predicting MI intention of the target subject.

As shown in FIG. 8, to test the feature extractor (that is, the target extraction model) and the classifier (that is, the target classification model) by using MI electroencephalogram signals in the test set, the MI electroencephalogram signals in the test set may be preprocessed first. For the specific implementation process of preprocessing, reference may be made to the relevant description in FIG. 5.

The method for processing an MI electroencephalogram signal through unsupervised learning may support unlabeled subject samples as the target domain to perform training, to obtain efficient and useful EEG decoding models, so as to make the application of BCIs more practical. According to another aspect of the embodiments of this application, a method for processing an MI electroencephalogram signal is further provided. Exemplarily, the above method for processing an MI electroencephalogram signal may be applied to, but is not limited to, the network architecture shown in FIG. 1.

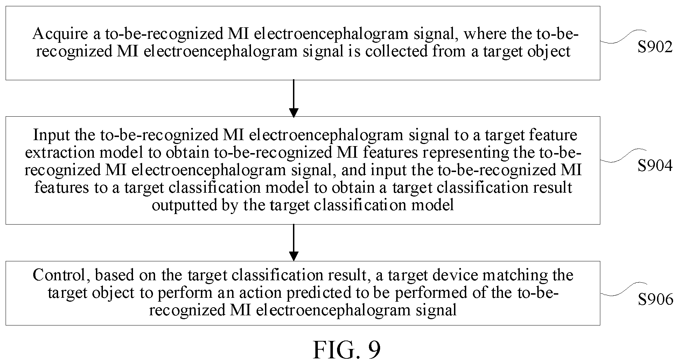

Exemplarily, in this embodiment, as an exemplary implementation, as shown in FIG. 9, the method for processing an MI electroencephalogram signal may include the following steps S902 to S906.

In step S902, a to-be-recognized MI electroencephalogram signal is acquired, where the to-be-recognized MI electroencephalogram signal is collected from a target object.

In step S904, the to-be-recognized MI electroencephalogram signal is inputted to a target feature extraction model, to obtain to-be-recognized MI features representing the to-be-recognized MI-EGG signal, and the to-be-recognized MI features is inputted to a target classification model, to obtain a target classification result outputted by the target classification model. A similarity between feature distributions of different MI features extracted by the target feature extraction model for different MI training signals is greater than or equal to a first similarity threshold, the different MI training signals include a target MI electroencephalogram signal and a source MI electroencephalogram signal corresponding to a first known action, an action predicted to be performed, represented by a classification result outputted by the target classification model, of the source MI electroencephalogram signal is consistent with the first known action,

the classification result is determined by the target classification model based on source MI features extracted from the source MI electroencephalogram signal by the target feature extraction model, and the target classification result represents an action predicted to be performed of the to-be-recognized MI electroencephalogram signal.

In step S906, a target device matching the target object is controlled based on the target classification result to perform the action predicted to be performed of the to-be-recognized MI electroencephalogram signal.

Exemplarily, the method for processing an MI electroencephalogram signal may be applied to, but is not limited to, the processes of transmission and control of ideas. For example, a BCI system combined with an exoskeleton robot may be used for active rehabilitation of motor functions of patients with hemiplegia or cerebral stroke, a BCI system combined with an electric wheelchair may help users with physical disabilities to travel freely, and a brain-computer-game system combined with a game may achieve activities of objects (including, but not limited to, at least one of the following: characters, articles, and the like) in a virtual world controlled by a human body through ideas and imagination.

In this embodiment, a type of an action of the to-be-recognized MI electroencephalogram signal is recognized by using the target feature extraction model and the target classification model. Further, different MI electroencephalogram signals extracted by the target feature extraction model have similar feature distributions, and the target classification model may correctly recognize the first known action corresponding to the source MI electroencephalogram signal based on the source MI feature, which solves the technical problem of [关键词], improving the generalization capability of the MI recognition model (including the obtained feature extraction model and classification model) and the accuracy of decoding.

Furthermore, end-to-end decoding and classification are performed on the motor imagery electroencephalogram signals through deep learning, and the decoding result may be obtained directly by inputting the original signal without too much prior knowledge of manual extraction of features, so that the model is more universal.

It is to be noted that, for ease of description, the foregoing method embodiments are described as a combination of a series of actions. However, a person skilled in the art is to learn that this application is not limited to the described sequence of the actions, according to this application, some steps may be performed in another sequence or may be simultaneously performed. In addition, a person skilled in the art is also to learn that the embodiments described in this specification are preferable, and the involved actions and modules are not necessary to this application.

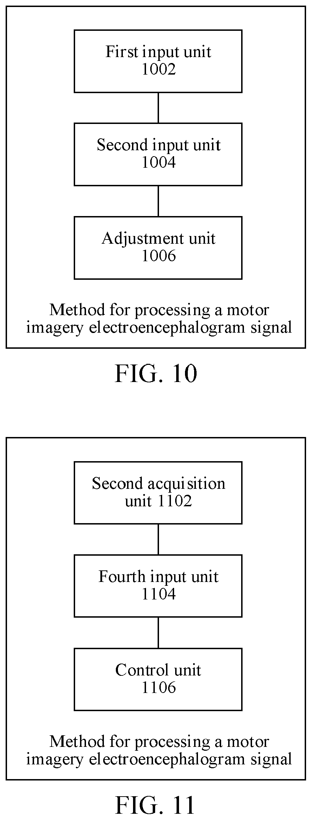

According to still another aspect of the embodiments of this application, an apparatus for processing an MI electroencephalogram signal used for implementing the above method for processing an MI electroencephalogram signal is further provided. As shown in FIG. 10, the apparatus includes: a first input unit 1002, a second input unit 1004, and an adjustment unit 1006.

(1) The first input unit 1002 is configured to input a source MI electroencephalogram signal belonging to a source domain and a target MI electroencephalogram signal belonging to a target domain to an initial feature extraction model, to obtain first source MI features representing the source MI electroencephalogram signal and first target MI features representing the target MI electroencephalogram signal.

(2) The second input unit 1004 is configured to input the first source MI features to an initial classification model, to obtain a first classification result outputted by the initial classification model. The first classification result represents an action predicted to be performed of the source MI electroencephalogram signal.

(3) The adjustment unit 1006 is configured to adjust a model parameter of the initial feature extraction model and/or a model parameter of the initial classification model to obtain a target feature extraction model and a target classification model in a case that a first known action corresponding to the source MI electroencephalogram signal is inconsistent with the action predicted to be performed, represented by the first classification result, of the source MI electroencephalogram signal, or a similarity between a feature distribution of the first source MI features and a feature distribution of the first target MI features is less than a first predetermined threshold. A similarity between a feature distribution of second source MI features representing the source MI electroencephalogram signal that are outputted by the target feature extraction model and a feature distribution of second target MI features representing the target MI electroencephalogram signal that are outputted by the target feature extraction model is greater than or equal to the first predetermined threshold, and an action predicted to be performed, represented by a second classification result outputted by the target classification model, of the source MI electroencephalogram signal is consistent with the first known action.

Exemplarily, the apparatus for processing an MI electroencephalogram signal may be applied to, but is not limited to, the processes of transmission and control of ideas. For example, a BCI system combined with an exoskeleton robot may be used for active rehabilitation of motor functions of patients with hemiplegia or cerebral stroke, a BCI system combined with an electric wheelchair may help users with physical disabilities to travel freely, and a brain-computer-game system combined with a game may achieve activities of objects (including, but not limited to, at least one of the following: characters, articles, and the like) in a virtual world controlled by a human body through ideas and imagination.

Exemplarily, the first input unit 1002 may be configured to perform the foregoing step S202, the second input unit 1004 may be configured to perform the foregoing step S204, and the adjustment unit 1006 may be configured to perform the foregoing step S206.

In this embodiment, the feature extraction model and the classification model are trained by using the source MI electroencephalogram signal corresponding to the first known action and the target MI electroencephalogram signal, so that the features of the source MI electroencephalogram signal and the features of the target MI electroencephalogram signal are extracted by the feature extraction model are distributed similarly, and the classification model can correctly recognize the first known action, which solves the technical problem of a low accuracy of decoding MI electroencephalogram signals of different objects by an MI recognition model due to the MI electroencephalogram signals varying greatly among subjects in the conventional art, and improves the generalization capability of the MI recognition model and the accuracy of decoding.

In an exemplary implementation, the adjustment unit 1006 includes: a first input module and an adjustment module.

(1) The first input module is configured to input the first source MI features and the first target MI features to an initial domain discrimination model, to obtain a first source determination result and a first target determination result. The initial domain discrimination model is configured to: determine, based on the feature distribution of the first source MI features, a probability that the source MI electroencephalogram signal belongs to the source domain and a probability that the source MI electroencephalogram signal belongs to the target domain, and determine, based on the feature distribution of the first target MI features, a probability that the target MI electroencephalogram signal belongs to the source domain and a probability that the target MI electroencephalogram signal belongs to the target domain.

(2) The adjustment module is configured to adjust the model parameter of the initial feature extraction model and/or the model parameter of the initial classification model, and a model parameter of the initial domain discrimination model by using the source MI electroencephalogram signal and the target MI electroencephalogram signal via multiple iterations, to obtain a target feature extraction model, a target classification model, and a target domain discrimination model. A difference between the probability that the source MI electroencephalogram signal belongs to the source domain and the probability that the source MI electroencephalogram signal belongs to the target domain that are determined by the target domain discrimination model based on the second source MI features is less than or equal to a second predetermined threshold, and a difference between the probability that the target MI electroencephalogram signal belongs to the source domain and the probability that the target MI electroencephalogram signal belongs to the target domain that are determined by the target domain discrimination model based on the second target MI features is less than or equal to the second predetermined threshold.

In an exemplary implementation, the above apparatus further includes: a third input unit, and a determination unit.

(1) The third input unit is configured to input, in a case that the target MI electroencephalogram signal corresponds to a second known action and before the adjusting a model parameter of the initial feature extraction model and/or a model parameter of the initial classification model to obtain a target feature extraction model and a target classification model, the first target MI features to the initial classification model to obtain a third classification result outputted by the initial classification model, the third classification result represents an action predicted to be performed of the target MI electroencephalogram signal.

(2) The determination unit is configured to trigger, in a case that the first known action is inconsistent with the action predicted to be performed of the source MI electroencephalogram signal, or that the second known action is inconsistent with the action predicted to be performed, represented by the third classification result, of the target MI electroencephalogram signal, or that a similarity between the feature distribution of the first source MI features and the feature distribution of the first target MI features is less than the first predetermined threshold, the adjustment unit 1006 to perform the step of adjusting the model parameter of the initial feature extraction model and/or the model parameter of the initial classification model, to obtain the target feature extraction model and the target classification model.

In an exemplary implementation, the above apparatus further includes: a first acquisition unit, a first preprocessing unit, and a second preprocessing unit.

(1) The first acquisition unit is configured to acquire an initial source MI electroencephalogram signal belonging to the source domain and an initial target MI electroencephalogram signal belonging to the target domain before the inputting a source MI electroencephalogram signal belonging to the source domain and a target MI electroencephalogram signal belonging to the target domain to an initial feature extraction model.

(2) The first preprocessing unit is configured to preprocess the initial source MI electroencephalogram signal to obtain the source MI electroencephalogram signal.

(3) The second preprocessing unit is configured to preprocess the initial target MI electroencephalogram signal to obtain the target MI electroencephalogram signal.

In this embodiment, the initial source MI electroencephalogram signal and the initial target MI electroencephalogram signal are preprocessed, so that the MI electroencephalogram signal can be optimized, thereby improving the effectiveness of training the MI electroencephalogram signal recognition model.

In an exemplary implementation, the first preprocessing unit includes: a first intercepting module, a first filtering module, and a first standardization module.

(1) The first intercepting module is configured to intercept a signal of a predetermined duration from the initial source MI electroencephalogram signal, to serve as a first source MI electroencephalogram signal.

(2) The first filtering module is configured to input the first source MI electroencephalogram signal to a band-pass filter, to obtain a second source MI electroencephalogram signal. The band-pass filter is configured to filter out, from the first source MI electroencephalogram signal, a signal not in a frequency band range of the band-pass filter.

(3) The first standardization module is configured to standardize the second source MI electroencephalogram signal to obtain the source MI electroencephalogram signal.

In an exemplary implementation, the second preprocessing unit includes: a second intercepting module, a second filtering module, and a second standardization module.

(1) The second intercepting module is configured to intercept a signal of a predetermined duration from the initial target MI electroencephalogram signal, to serve as a first target MI electroencephalogram signal.

(2) The second filtering module is configured to input the first target MI electroencephalogram signal to a band-pass filter, to obtain a second target MI electroencephalogram signal. The band-pass filter is configured to filter out, from the first target MI electroencephalogram signal, a signal not in a band-pass frequency band range.

(3) The second standardization module is configured to standardize the second target MI electroencephalogram signal to obtain the target MI electroencephalogram signal.

In this embodiment, the initial MI electroencephalogram signal is preprocessed to filter out noise data, and lengths of the MI electroencephalogram signals are unified and the MI electroencephalogram signals are standardized, thereby improving the effectiveness of training the MI electroencephalogram signal recognition model.
In an exemplary implementation, the above apparatus further includes: a transmission unit, and a receiving unit.

(1) The transmission unit is configured to transmit a target instruction to a collection device before the source MI electroencephalogram signal belonging to the source domain and the target MI electroencephalogram signal belonging to the target domain are inputted to the initial feature extraction model, where the target instruction indicates a to-be-performed first known action.

(2) The receiving unit is configured to receive the source MI electroencephalogram signal transmitted from the collection device, where the source MI electroencephalogram signal is collected by the collection device within a preset time period after receiving the target instruction.

In this embodiment, the collection device is instructed to collect MI electroencephalogram signals in response to the target instruction indicating the to-be-performed first known action, so that the source MI electroencephalogram signal corresponding to the first known action can be obtained, thereby ensuring the accuracy of acquiring the source MI electroencephalogram signal.

According to still another aspect of the embodiments of this application, an apparatus for processing an MI electroencephalogram signal used for implementing the above method for processing an MI electroencephalogram signal is further provided. As shown in FIG. 11, the apparatus includes: a second acquisition unit 1102, a fourth input unit 1104, and a control unit 1106.

(1) The second acquisition unit 1102 is configured to acquire a to-be-recognized MI electroencephalogram signal, where the to-be-recognized MI electroencephalogram signal is collected from a target object.

(2) The fourth input unit 1104 is configured to successively input the to-be-recognized MI electroencephalogram signal to a target feature extraction model, to obtain to-be-recognized MI features representing the to-be-recognized MI-EGG signal, and input the to-be-recognized MI features to a target classification model, to obtain a target classification result outputted by the target classification model. A similarity between feature distributions of different MI features extracted by the target feature extraction model for different MI training signals is greater than or equal to a first similarity threshold, the different MI training signals include a target MI electroencephalogram signal and a source MI electroencephalogram signal corresponding to a first known action, an action predicted to be performed, represented by a classification result outputted by the target classification model, of the source MI electroencephalogram signal is consistent with the first known action, the classification result is determined by the target classification model based on source MI features extracted from the source MI electroencephalogram signal by the target feature extraction model, and the target classification result represents an action predicted to be performed of the to-be-recognized MI electroencephalogram signal.

(3) The control unit 1106 is configured to control, based on the target classification result, a target device matching the target object to perform the action predicted to be performed of the to-be-recognized MI electroencephalogram signal.

Exemplarily, the apparatus for processing an MI electroencephalogram signal may be applied to, but is not limited to, the processes of transmission and control of ideas. For example, a BCI system combined with an exoskeleton robot may be used for active rehabilitation of motor functions of patients with hemiplegia or cerebral stroke, a BCI system combined with an electric wheelchair may help users with physical disabilities to travel freely, and a brain-computer-game system combined with a game may achieve activities of objects (including, but not limited to, at least one of the following: characters, articles, and the like) in a virtual world controlled by a human body through ideas and imagination.
Exemplarily, the second acquisition unit 1102 may be configured to perform the foregoing step S902, the fourth input unit 1104 may be configured to perform the foregoing step S904, and the control unit 1106 may be configured to perform the foregoing step S906.
In this embodiment, a type of an action of the to-be-recognized MI electroencephalogram signal is recognized by using the target feature extraction model and the target classification model. Further, different MI electroencephalogram signals extracted by the target feature extraction model have similar feature distributions, and the target classification model may correctly recognize the first known action corresponding to the source MI electroencephalogram signal based on the source MI feature, which solves the technical problem of a low accuracy of decoding MI electroencephalogram signals of different objects by an MI recognition model due to the MI electroencephalogram signals varying greatly among subjects in the conventional art, and improves the generalization capability of the MI recognition model (including the obtained feature extraction model and classification model) and the accuracy of decoding.
It is to be noted that each of the above modules may be implemented by software or hardware. For hardware, the modules may be implemented in the following way, which is not limited to: the above modules are all located in the same processor; or the above modules are located in different processors in the form of any combinations.
According to still another aspect of the embodiments of this application, a storage medium is further provided, which stores a computer program. The computer program is configured to perform, when run, steps in any one of the foregoing method embodiments.
Exemplarily, in this embodiment, the storage medium may be configured to store a computer program for performing the following steps S1 to S3.
In step S1, a source MI electroencephalogram signal belonging to a source domain and a target MI electroencephalogram signal belonging are inputted to a target domain to an initial feature extraction model, to obtain first source MI features representing the source MI electroencephalogram signal and first target MI features representing the target MI electroencephalogram signal.
In step S2, the first source MI features are inputted to an initial classification model, to obtain a first classification result outputted by the initial classification model. The first classification result represents an action predicted to be performed of the source MI electroencephalogram signal.
In step S3, a model parameter of the initial feature extraction model and/or a model parameter of the initial classification model is adjusted to obtain a target feature extraction model and a target classification model in a case that a first known action corresponding to the source MI electroencephalogram signal is inconsistent with the action predicted to be performed, represented by the first classification result, of the source MI electroencephalogram signal, or a similarity between a feature distribution of the first source MI features and a feature distribution of the first target MI features is less than a first predetermined threshold. A similarity between a feature distribution of second source MI features representing the source MI electroencephalogram signal that are outputted by the target feature extraction model and a feature distribution of second target MI features representing the target MI electroencephalogram signal that are outputted by the target feature extraction model is greater than or equal to the first predetermined threshold, and an action predicted to be performed, represented by a second classification result outputted by the target classification model, of the source MI electroencephalogram signal is consistent with the first known action.

In an embodiment, the following steps are performed.

In step S1', a to-be-recognized MI electroencephalogram signal is acquired, where the to-be-recognized MI electroencephalogram signal is collected from a target object.

In step S2', the to-be-recognized MI electroencephalogram signal is inputted to a target feature extraction model, to obtain to-be-recognized MI features representing the to-be-recognized MI-EGG signal, and input the to-be-recognized MI features to a target classification model, to obtain a target classification result outputted by the target classification model. A similarity between feature distributions of different MI features extracted by the target feature extraction model for different MI training signals is greater than or equal to a first similarity threshold, the different MI training signals include a target MI electroencephalogram signal and a source MI electroencephalogram signal corresponding to a first known action, an action predicted to be performed, represented by a classification result outputted by the target classification model, of the source MI electroencephalogram signal is consistent with the first known action, the classification result is determined by the target classification model based on source MI features extracted from the source MI electroencephalogram signal by the target feature extraction model, and the target classification result represents an action predicted to be performed of the to-be-recognized MI electroencephalogram signal.

In step S3', a target device matching the target object is controlled based on the target classification result to perform the action predicted to be performed of the to-be-recognized MI electroencephalogram signal.

Exemplarily, in this embodiment, the storage medium may be configured to store a computer program for performing the following steps.

Exemplarily, in this embodiment, a person of ordinary skill in the art may understand that all or some of the steps of the methods in the foregoing embodiments may be implemented by a program indicating relevant hardware of the terminal device. The program may be stored in a computer-readable storage medium. The storage medium may include a flash disk, a read-only memory (ROM), a random access memory (RAM), a magnetic disk, an optical disc, and the like.

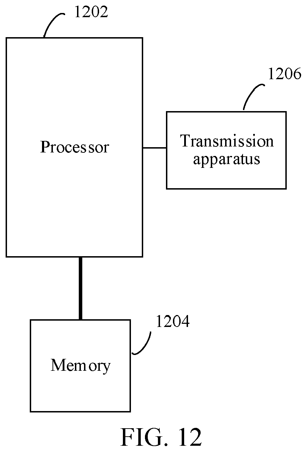

According to still another aspect of the embodiments of this application, an electronic device configured to perform the method for processing an MI electroencephalogram signal is further provided. As shown in FIG. 12, the electronic device may include: a processor 1202, a memory 1204, a transmission apparatus 1206, and the like. The memory stores a computer program, and the processor is configured to perform steps in any one of the method embodiments through the computer program.

Exemplarily, in this embodiment, the electronic device may be located in at least one of multiple network devices in a computer network.

Exemplarily, in this embodiment, the processor may be configured to execute a computer program to perform the following steps S1 to S3.

In step S1, a source MI electroencephalogram signal belonging to a source domain and a target MI electroencephalogram signal belonging are inputted to a target domain to an initial feature extraction model, to obtain first source MI features representing the source MI electroencephalogram signal and first target MI features representing the target MI electroencephalogram signal.

In step S2, the first source MI features are inputted to an initial classification model, to obtain a first classification result outputted by the initial classification model. The first classification result represents an action predicted to be performed of the source MI electroencephalogram signal.

In step S3, a model parameter of the initial feature extraction model and/or a model parameter of the initial classification model is adjusted to obtain a target feature extraction model and a target classification model in a case that a first known action corresponding to the source MI electroencephalogram signal is inconsistent with the action predicted to be performed, represented by the first classification result, of the source MI electroencephalogram signal, or a similarity between a feature distribution of the first source MI features and a feature distribution of the first target MI features is less than a first predetermined threshold. A similarity between a feature distribution of second source MI features representing the source MI electroencephalogram signal that are outputted by the target feature extraction model and a feature distribution of second target MI features representing the target MI electroencephalogram signal that are outputted by the target feature extraction model is greater than or equal to the first predetermined threshold, and an action predicted to be performed, represented by a second classification result outputted by the target classification model, of the source MI electroencephalogram signal is consistent with the first known action.

In an embodiment, the following steps are performed.

In step S1', a to-be-recognized MI electroencephalogram signal is acquired, where the to-be-recognized MI electroencephalogram signal is collected from a target object.

In step S2', the to-be-recognized MI electroencephalogram signal is inputted to a target feature extraction model, to obtain to-be-recognized MI features representing the to-be-recognized MI-EGG signal, and input the to-be-recognized MI features to a target classification model, to obtain a target classification result outputted by the target classification model. A similarity between feature distributions of different MI features extracted by the target feature extraction model for different MI training signals is greater than or equal to a first similarity threshold, the different MI training signals include a target MI electroencephalogram signal and a source MI electroencephalogram signal corresponding to a first known action, an action predicted to be performed, represented by a classification result outputted by the target classification model, of the source MI electroencephalogram signal is consistent with the first known action, the classification result is determined by the target classification model based on source MI features extracted from the source MI electroencephalogram signal by the target feature extraction model, and the target classification result represents an action predicted to be performed of the to-be-recognized MI electroencephalogram signal.

In step S3', a target device matching the target object is controlled based on the target classification result, to perform the action predicted to be performed of the to-be-recognized MI electroencephalogram signal.

Exemplarily, in this embodiment, the processor may be configured to store a computer program for performing the following steps.

Exemplarily, a person of ordinary skill in the art may understand that the structure shown in FIG. 12 is only an example. The electronic device may alternatively be a server, an MI electroencephalogram signal collection device, or the like. FIG. 12 does not constitute a limitation on the structure of the electronic device. For example, the electronic device may further include more or fewer components (such as a network interface) than those shown in FIG. 12, or have a configuration different from that shown in FIG. 12.

The memory 1204 may be configured to store a software program and module, for example, a program instruction/module corresponding to the method and apparatus for processing an MI electroencephalogram signal in the embodiments of this application. The processor 1202 runs the software program and module stored in the memory 1204, to implement various functional applications and data processing, that is, implement the foregoing method for processing an MI electroencephalogram signal. The memory 1204 may include a high-speed random memory, and may also include a non-volatile memory, for example, one or more magnetic storage apparatuses, a flash memory, or another non-volatile solid-state memory. In some examples, the memory 1204 may further include memories remotely disposed relative to the processor 1202, and the remote memories may be connected to a terminal through a network. The foregoing examples of the network include, but are not limited to, the Internet, an intranet, a local area network, a mobile communication network, and a combination thereof.

A transmission device 1206 is configured to receive or transmit data through a network. Specific examples of the foregoing network may include a wired network and a wireless network. In an example, the transmission apparatus 1206 includes a network interface controller (NIC). The NIC may be connected to another network device and a router by using a network cable, to communicate with the Internet or a local area network. In an example, the transmission apparatus 1206 is a radio frequency (RF) module, which communicates with the Internet in a wireless manner.

The sequence numbers of the foregoing embodiments of this application are merely for description purpose, and are not intended to indicate the preference among the embodiments.

In a case that the integrated unit in the foregoing embodiments is implemented in the form of a software function unit and sold or used as an independent product, the integrated unit may be stored in the foregoing computer-readable storage medium. Based on such an understanding, the technical solutions of this application essentially, or a part contributing to the related art, or all or a part of the technical solution may be implemented in a form of a software product. The computer software product is stored in a storage medium and includes several instructions for instructing one or more computer devices (which may be a PC, a server, a network device, or the like) to perform all or some of steps of the methods in the embodiments of this application.

In the foregoing embodiments of this application, descriptions of the embodiments have different emphases. As for parts that are not described in detail in one embodiment, reference may be made to the relevant descriptions of the other embodiments.

In the several embodiments provided in this application, it is to be understood that the disclosed client may be implemented in other manners. The described apparatus embodiments are merely exemplary. For example, the unit division is merely logical function division, and may use other division manners during actual implementation. For example, multiple units or components may be combined or integrated into another system, or some features may be omitted or not performed. In addition, the coupling, or direct coupling, or communication connection between the displayed or discussed components may be the indirect coupling or communication connection by using some interfaces, units, or modules, and may be electrical or of other forms.

The units described as separate parts can or cannot be physically separate. Parts displayed as units can or cannot be physical units, and can be located in one position, or can be distributed on multiple network units. Some or all of the units may be selected according to actual needs to achieve the objectives of the solutions of the embodiments.

In addition, functional units in the embodiments of this application may be integrated into one processing unit, or each of the units may exist alone physically, or two or more units may be integrated into one unit. The integrated unit may be implemented in a form of hardware, or may be implemented in a form of a software functional unit.

The foregoing descriptions are merely exemplary implementations of this application. A person of ordinary skill in the art may make some improvements and polishing without departing from the principle of this application and the improvements and polishing shall fall within the protection scope of this application.

**Claims**

1. A method for processing a motor imagery electroencephalogram signal, performed by an electronic device, the method comprising:

   inputting a source motor imagery, MI, electroencephalogram signal belonging to a source domain and a target MI electroencephalogram signal belonging to a target domain to an initial feature extraction model, to obtain first source MI features representing the source MI electroencephalogram signal and first target MI features representing the target MI electroencephalogram signal;
   inputting the first source MI features to an initial classification model, to obtain a first classification result outputted by the initial classification model, the first classification result representing an action predicted to be performed of the source MI electroencephalogram signal; and
   adjusting a model parameter of the initial feature extraction model and/or a model parameter of the initial classification model to obtain a target feature extraction model and a target classification model, in a case that a first known action corresponding to the source MI electroencephalogram signal is inconsistent with the action predicted to be performed, represented by the first classification result, of the source MI electroencephalogram signal, or a similarity between a feature distribution of the first source MI features and a feature distribution of the first target MI features is less than a first predetermined threshold, wherein
   a similarity between a feature distribution of second source MI features representing the source MI electroencephalogram signal that are outputted by the target feature extraction model and a feature distribution of second target MI features representing the target MI electroencephalogram signal that are outputted by the target feature extraction model is greater than or equal to the first predetermined threshold, and an action predicted to be performed, represented by a second classification result outputted by the target classification model, of the source MI electroencephalogram signal that is consistent with the first known action.

2. The method according to claim 1, wherein the adjusting a model parameter of the initial feature extraction model and/or a model parameter of the initial classification model to obtain a target feature extraction model and a target classification model comprises:

   inputting the first source MI features and the first target MI features to an initial domain discrimination model, to obtain a first source determination result and a first target determination result, the initial domain discrimination model being configured to: determine, based on the feature distribution of the first source MI features, a probability that the source MI electroencephalogram signal belongs to the source domain and a probability that the source MI electroencephalogram signal belongs to the target domain, and determine, based on the feature distribution of the first target MI features, a probability that the target MI electroencephalogram signal belongs to the source domain and a probability that the target MI electroencephalogram signal belongs to the target domain; and
   adjusting the model parameter of the initial feature extraction model and/or the model parameter of the initial classification model and a model parameter of the initial domain discrimination model by using the source MI electroencephalogram signal and the target MI electroencephalogram signal via a plurality of iterations, to obtain the target feature extraction model, the target classification model, and a target domain discrimination model, wherein
   a difference between the probability that the source MI electroencephalogram signal belongs to the source domain and the probability that the source MI electroencephalogram signal belongs to the target domain that are determined by the target domain discrimination model based on the second source MI features is less than or equal to a second predetermined threshold, and a difference between the probability that the target MI electroencephalogram signal belongs to the source domain and the probability that the target MI electroencephalogram signal belongs to the target domain that are determined by the target domain discrimination model based on the second target MI features is less than or equal to the second predetermined threshold.

3. The method according to claim 1, wherein in a case that the target MI electroencephalogram signal corresponds to a second known action, before the adjusting a model parameter of the initial feature extraction model and/or a model parameter of the initial classification model to obtain a target feature extraction model and a target classification model, the method further comprises:

inputting the first target MI features to the initial classification model to obtain a third classification result outputted by the initial classification model, the third classification result representing an action predicted to be performed of the target MI electroencephalogram signal, wherein
the step of adjusting the model parameter of the initial feature extraction model and/or the model parameter of the initial classification model to obtain the target feature extraction model and the target classification model is performed in a case that the first known action is inconsistent with the action predicted to be performed of the source MI electroencephalogram signal, or that the second known action is inconsistent with the action predicted to be performed, represented by the third classification result, of the target MI electroencephalogram signal that, or that a similarity between the feature distribution of the first source MI features and the feature distribution of the first target MI features is less than the first predetermined threshold.

4. The method according to claim 1, wherein before the inputting a source MI electroencephalogram signal belonging to a source domain and a target MI electroencephalogram signal belonging to a target domain to an initial feature extraction model, the method further comprises:

acquiring an initial source MI electroencephalogram signal belonging to the source domain and an initial target MI electroencephalogram signal belonging to the target domain;
preprocessing the initial source MI electroencephalogram signal to obtain the source MI electroencephalogram signal; and
preprocessing the initial target MI electroencephalogram signal to obtain the target MI electroencephalogram signal.

5. The method according to claim 4, wherein the preprocessing the initial source MI electroencephalogram signal to obtain the source MI electroencephalogram signal comprises:

intercepting a signal of a predetermined duration from the initial source MI electroencephalogram signal, to serve as a first source MI electroencephalogram signal;
inputting the first source MI electroencephalogram signal to a band-pass filter, to obtain a second source MI electroencephalogram signal, the band-pass filter being configured to filter out, from the first source MI electroencephalogram signal, a signal not in a band-pass frequency band; and
standardizing the second source MI electroencephalogram signal to obtain the source MI electroencephalogram signal.

6. The method according to claim 4, wherein the preprocessing the initial target MI electroencephalogram signal, to obtain the target MI electroencephalogram signal comprises:

intercepting a signal of a predetermined duration from the initial target MI electroencephalogram signal, to serve as a first target MI electroencephalogram signal;
inputting the first target MI electroencephalogram signal to a band-pass filter, to obtain a second target MI electroencephalogram signal, the band-pass filter being configured to filter out, from the first target MI electroencephalogram signal, a signal not in a band-pass frequency band; and
standardizing the second target MI electroencephalogram signal to obtain the target MI electroencephalogram signal.

7. The method according to any one of claims 1 to 6, wherein before the inputting a source MI electroencephalogram signal belonging to a source domain and a target MI electroencephalogram signal belonging to a target domain to an initial feature extraction model, the method further comprises:

transmitting a target instruction to a collection device, the target instruction indicating the first known action to be performed; and

receiving a source MI electroencephalogram signal transmitted from the collection device, the source MI electroencephalogram signal being collected by the collection device within a preset time period after receiving the target instruction.

8. A method for processing a motor imagery electroencephalogram signal, performed by an electronic device, the method comprising:

acquiring a to-be-recognized motor imagery, MI, electroencephalogram signal, the to-be-recognized MI electroencephalogram signal being collected from a target object;
inputting the to-be-recognized MI electroencephalogram signal to a target feature extraction model, to obtain to-be-recognized MI features representing the to-be-recognized MI electroencephalogram signal, and inputting the to-be-recognized MI features to a target classification model, to obtain a target classification result outputted by the target classification model, wherein

a similarity between feature distributions of different MI features extracted by the target feature extraction model for different MI training signals is greater than or equal to a first similarity threshold,
the different MI training signals comprises a target MI electroencephalogram signal and a source MI electroencephalogram signal corresponding to a first known action,
an action predicted to be performed, represented by a classification result outputted by the target classification model, of the source MI electroencephalogram signal is consistent with the first known action,
the classification result is determined by the target classification model based on source MI features extracted from the source MI electroencephalogram signal by the target feature extraction model, and the target classification result represents an action predicted to be performed of the to-be-recognized MI electroencephalogram signal; and
controlling, based on the target classification result, a target device matching the target object to perform the action predicted to be performed of the to-be-recognized MI electroencephalogram signal.

9. An apparatus for processing a motor imagery electroencephalogram signal, comprising:

a first input unit, configured to input a source motor imagery, MI, electroencephalogram signal belonging to a source domain and a target MI electroencephalogram signal belonging to a target domain to an initial feature extraction model, to obtain first source MI features representing the source MI electroencephalogram signal and first target MI features representing the target MI electroencephalogram signal;
a second input unit, configured to input the first source MI features to an initial classification model, to obtain a first classification result outputted by the initial classification model, the first classification result representing an action predicted to be performed of the source MI electroencephalogram signal; and
an adjustment unit, configured to adjust a model parameter of the initial feature extraction model and/or a model parameter of the initial classification model to obtain a target feature extraction model and a target classification model, in a case that a first known action corresponding to the source MI electroencephalogram signal is inconsistent with the action predicted to be performed, represented by the first classification result, of the source MI electroencephalogram signal, or a similarity between a feature distribution of the first source MI features and a feature distribution of the first target MI features is less than a first predetermined threshold, wherein a similarity between a feature distribution of second source MI features representing the source MI electroencephalogram signal that are outputted by the target feature extraction model and a feature distribution of second target MI features representing the target MI electroencephalogram signal that are outputted by the target feature extraction model is greater than or equal to the first predetermined threshold, and an action predicted to be performed, represented by a second classification result outputted by the target classification model, of the source MI electroencephalogram signal is consistent with the first known action.

10. The apparatus according to claim 9, wherein the adjustment unit comprises:

a first input module, configured to input the first source MI features and the first target MI features to an initial domain discrimination model, to obtain a first source determination result and a first target determination result, the initial domain discrimination model being configured to: determine, based on the feature distribution of the first source MI features, a probability that the source MI electroencephalogram signal belongs to the source domain and a probability that the source MI electroencephalogram signal belongs to the target domain, and determine, based on the feature distribution of the first target MI features, a probability that the target MI electroencephalogram signal belongs to the source domain and a probability that the target MI electroencephalogram signal belongs to the target domain; and

an adjustment module, configured to adjust the model parameter of the initial feature extraction model and/or the model parameter of the initial classification model and a model parameter of the initial domain discrimination model by using the source MI electroencephalogram signal and the target MI electroencephalogram signal via a plurality of iterations, to obtain the target feature extraction model, the target classification model, and a target domain discrimination model, wherein

a difference between the probability that the source MI electroencephalogram signal belongs to the source domain and the probability that the source MI electroencephalogram signal belongs to the target domain that are determined by the target domain discrimination model based on the second source MI features is less than or equal to a second predetermined threshold, and a difference between the probability that the target MI electroencephalogram signal belongs to the source domain and the probability that the target MI electroencephalogram signal belongs to the target domain that are determined by the target domain discrimination model based on the second target MI features is less than or equal to the second predetermined threshold.

**11.** The apparatus according to claim 9, further comprising:

a third input unit, configured to input, in a case that the target MI electroencephalogram signal corresponds to a second known action and before the model parameter of the initial feature extraction model and/or the model parameter of the initial classification model are adjusted to obtain the target feature extraction model and the target classification model, the first target MI features to the initial classification model to obtain a third classification result outputted by the initial classification model, the third classification result representing an action predicted to be performed of the target MI electroencephalogram signal; and

a determination unit, configured to trigger the adjustment unit to perform the step of adjusting the model parameter of the initial feature extraction model and/or the model parameter of the initial classification model to obtain the target feature extraction model and the target classification model in a case that the first known action is inconsistent with the action predicted to be performed of the source MI electroencephalogram signal, or that the second known action is inconsistent with the action predicted to be performed, represented by the third classification result, of the target MI electroencephalogram signal, or that a similarity between the feature distribution of the first source MI features and the feature distribution of the first target MI features is less than the first predetermined threshold.

**12.** The apparatus according to any one of claims 9 to 11, further comprising:

a first acquisition unit, configured to acquire an initial source MI electroencephalogram signal belonging to the source domain and an initial target MI electroencephalogram signal belonging to the target domain before a source MI electroencephalogram signal belonging to the source domain and a target MI electroencephalogram signal belonging to the target domain are inputted to an initial feature extraction model;

a first preprocessing unit, configured to preprocess the initial source MI electroencephalogram signal to obtain the source MI electroencephalogram signal; and

a second preprocessing unit, configured to preprocess the initial target MI electroencephalogram signal to obtain the target MI electroencephalogram signal.

**13.** An apparatus for processing a motor imagery electroencephalogram signal, comprising:

a second acquisition unit, configured to acquire a to-be-recognized motor imagery, MI, electroencephalogram signal, the to-be-recognized MI electroencephalogram signal being collected from a target object;

a fourth input unit, configured to input the to-be-recognized MI electroencephalogram signal to a target feature extraction model, to obtain to-be-recognized MI features representing the to-be-recognized MI electroencephalogram signal, and input the to-be-recognized MI features to a target classification model, to obtain a target classification result outputted by the target classification model, wherein

a similarity between feature distributions of different MI features extracted by the target feature extraction model for different MI training signals is greater than or equal to a first similarity threshold,

the different MI training signals comprising a target MI electroencephalogram signal and a source MI electroencephalogram signal corresponding to a first known action,

an action predicted to be performed, represented by a classification result outputted by the target classification model, of the source MI electroencephalogram signal is consistent with the first known action, and

the classification result is determined by the target classification model based on source MI features extracted from the source MI electroencephalogram signal by the target feature extraction model, and the target classification result represents an action predicted to be performed of the to-be-recognized MI electroencephalogram signal; and

a control unit, configured to control, based on the target classification result, a target device matching the target object to perform the action predicted to be performed of the to-be-recognized MI electroencephalogram signal.

14. A computer-readable storage medium, storing a computer program, the computer program being configured to, when run, perform the method according to any one of claims 1 to 8.

15. An electronic device, comprising a memory and a processor, the memory storing a computer program, and the processor being configured to, through the computer program, perform the method according to any one of claims 1 to 8.

16. A computer program product, comprising instructions, the instructions, when run on a computer, causing the computer to perform the method according to any one of claims 1 to 8.

102

Terminal device

104

Network

106

...

FIG. 1

Input a source MI electroencephalogram signal belonging to a source domain and a target MI electroencephalogram signal belonging to a target domain to an initial feature extraction model, to obtain first source MI features representing the source MI-EEG signal and first target MI features representing the target MI electroencephalogram signal — S202

Input the first source MI features to an initial classification model, to obtain a first classification result outputted by the initial classification model, the first classification result representing an action predicted to be performed of the source MI electroencephalogram signal — S204

Adjust a model parameter of the initial feature extraction model and/or a model parameter of the initial classification model to obtain a target feature extraction model and a target classification model in a case that a first known action corresponding to the source MI electroencephalogram signal is inconsistent with the action predicted to be performed, represented by the first classification result, of the source MI electroencephalogram signal, or a similarity between a feature distribution of the first source MI features and a feature distribution of the first target MI features is less than a first predetermined threshold — S206

FIG. 2

Convolutional layer
(Time)
40 unit

44  ⌇25⌇

534

Convolutional layer
(all electrodes/patches)
40 unit

44  40  44

40

510

Square

Average pooling layer
Step size 15×1

40  75  1

510

Log

Linear classification layer
(Compact layer + Softmax)
4 unit

30  40

Hand (left)  Hand (right)  Foot  Others

FIG. 3

| Conversion pool module 1 | Convolutional layer<br>(Time)<br>25 Linear unit |  |
| | Spatial filter<br>(All electrodes, all<br>previous filters)<br>25 Exponential linear unit |  |
| | Max pooling layer<br>Step size 3×1 |  |

| Conversion pool<br>module 2 | Convolutional layer<br>50 Exponential linear unit |  |
| | Max pooling layer<br>Step size 3×1 |  |

| Conversion pool<br>module 3 | Convolutional layer<br>100 Exponential linear unit |  |
| | Max pooling layer<br>Step size 3×1 |  |

| Conversion pool<br>module 4 | Convolutional layer<br>200 Exponential linear unit |  |
| | Max pooling layer<br>Step size 3×1 |  |

| Classification layer | Linear classification layer<br>(Compact layer)<br>4 Softmax unit |  |

Hand (left)  Hand (right)  Foot  Others

FIG. 4

FIG. 5

FIG. 6

FIG. 7

FIG. 8

Acquire a to-be-recognized MI electroencephalogram signal, where the to-be-recognized MI electroencephalogram signal is collected from a target object

S902

Input the to-be-recognized MI electroencephalogram signal to a target feature extraction model to obtain to-be-recognized MI features representing the to-be-recognized MI electroencephalogram signal, and input the to-be-recognized MI features to a target classification model to obtain a target classification result outputted by the target classification model

S904

Control, based on the target classification result, a target device matching the target object to perform an action predicted to be performed of the to-be-recognized MI electroencephalogram signal

S906

FIG. 9

First input unit
1002

Second input unit
1004

Adjustment unit
1006

Method for processing a motor
imagery electroencephalogram signal

FIG. 10

Second acquisition
unit 1102

Fourth input unit
1104

Control unit
1106

Method for processing a motor
imagery electroencephalogram signal

FIG. 11

1202

Processor

1206

Transmission apparatus

1204

Memory

FIG. 12

## INTERNATIONAL SEARCH REPORT

| | International application No. |
|---|---|
| | **PCT/CN2020/112766** |

**A.    CLASSIFICATION OF SUBJECT MATTER**

G06F 3/01(2006.01)i;  G06K 9/00(2006.01)n;  G06N 3/04(2006.01)n;  A61B 5/0476(2006.01)n

According to International Patent Classification (IPC) or to both national classification and IPC

**B.    FIELDS SEARCHED**

Minimum documentation searched (classification system followed by classification symbols)

G06F; G06K; G06N; A61B

Documentation searched other than minimum documentation to the extent that such documents are included in the fields searched

Electronic data base consulted during the international search (name of data base and, where practicable, search terms used)

CNPAT, WPI, EPODOC, CNKI, IEEE: 运动想象, 脑电, 特征提取, 分类, 模型, 动作, 分布, 相似度, motor imagery, feature, extraction, electroencephalogram, EEG, classification, model, distribution, similarity

**C.    DOCUMENTS CONSIDERED TO BE RELEVANT**

| Category* | Citation of document, with indication, where appropriate, of the relevant passages | Relevant to claim No. |
|---|---|---|
| PX | CN 110531861 A (TENCENT TECHNOLOGY SHENZHEN CO., LTD.) 03 December 2019 (2019-12-03)<br>claims 1-15 | 1-16 |
| X | 韩飞 (HAN, Fei ). "基于运动想象的脑电特征提取及特征迁移方法研究 (Research on Feature Extraction and Feature Transfer of Eeg Based on Motor Imagery)"<br>中国优秀硕士学位论文全文数据库 医药卫生科技辑 (Chinese Master's Theses Full-text Database, Medical and Health Sciences), 15 February 2018 (2018-02-15), pp. 20-58 | 1-16 |
| A | CN 109583346 A (QILU UNIVERSITY OF TECHNOLOGY et al.) 05 April 2019 (2019-04-05)<br>entire document | 1-16 |
| A | CN 105468143 A (TIANJIN UNIVERSITY) 06 April 2016 (2016-04-06)<br>entire document | 1-16 |
| A | US 2019209038 A1 (HOLLAND BLOORVIEW KIDS REHABILITATION HOSPITAL) 11 July 2019 (2019-07-11)<br>entire document | 1-16 |

☐ Further documents are listed in the continuation of Box C.    ☑ See patent family annex.

| | |
|---|---|
| *    Special categories of cited documents:<br>"A"    document defining the general state of the art which is not considered to be of particular relevance<br>"E"    earlier application or patent but published on or after the international filing date<br>"L"    document which may throw doubts on priority claim(s) or which is cited to establish the publication date of another citation or other special reason (as specified)<br>"O"    document referring to an oral disclosure, use, exhibition or other means<br>"P"    document published prior to the international filing date but later than the priority date claimed | "T"    later document published after the international filing date or priority date and not in conflict with the application but cited to understand the principle or theory underlying the invention<br>"X"    document of particular relevance; the claimed invention cannot be considered novel or cannot be considered to involve an inventive step when the document is taken alone<br>"Y"    document of particular relevance; the claimed invention cannot be considered to involve an inventive step when the document is combined with one or more other such documents, such combination being obvious to a person skilled in the art<br>"&"    document member of the same patent family |

| Date of the actual completion of the international search | Date of mailing of the international search report |
|---|---|
| **26 October 2020** | **09 December 2020** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

INTERNATIONAL SEARCH REPORT
Information on patent family members

International application No.

**PCT/CN2020/112766**

| Patent document cited in search report | | | Publication date (day/month/year) | Patent family member(s) | | | Publication date (day/month/year) |
|---|---|---|---|---|---|---|---|
| CN | 110531861 | A | 03 December 2019 | None | | | |
| CN | 109583346 | A | 05 April 2019 | None | | | |
| CN | 105468143 | A | 06 April 2016 | WO | 2017084416 | A1 | 26 May 2017 |
| US | 2019209038 | A1 | 11 July 2019 | CA | 3087786 | A1 | 18 July 2019 |
| | | | | WO | 2019136555 | A1 | 18 July 2019 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201910843985